# EUROPEAN PATENT APPLICATION

(11) **EP 3 673 868 A1**
(43) Date of publication of application: **01.07.2020**
(21) Application number: 18001001.9
(22) Date of filing: 31.12.2018
(51) Int. Cl.: A61F 2/08, A61F 5/56

(54) **OBTURATOR FOR COVERING A DEFECT OF THE HARD PALATE AND SOFT PALATE**

(71) Applicant: Medizinische Universität Wien, 1090 Wien (AT)
(72) Inventor: UNGER, Ewald, 1210 Vienna (AT); OBEROI, Gunpreet, 1180 Vienna (AT)
(74) Representative: Keschmann, Marc

(57) **Abstract**

An obturator for covering a defect of the hard palate and soft palate, wherein the obturator comprises a hard palate-replacing rigid plate for covering the defect of the hard palate, is characterized by an inflatable, in particular semi-inflated soft member with a flexible outer shell arranged adjacent to the rigid plate and having a soft palate-replacing portion extending beyond a palate bone-replacing end of the rigid plate. The inventive palate implant is made of a biocompatible collagen pad or membrane for covering a defect of the soft palate, wherein the pad or membrane at least in a boundary area thereof has a reinforcement structure of a biocompatible filament.

## Description

The present invention relates to an obturator for covering a defect of the hard palate and soft palate. The present invention further relates to a palate implant.

Cleft palate and cleft lip is a common craniofacial disorder requiring a multidisciplinary treatment. From infancy to adulthood, patients face a plethora of problems including aesthetic compromise, velopharyngeal insufficiency causing dysarthria and hyper-nasality of speech, nasal emission of air and regurgitation and occlusal malformations, degeneration in palatal musculature causing impaired Eustachian tube patency, middle ear pressure imbalance, chronic otitis media with effusion and conductive hearing loss. Surgical correction of the palatal defect followed by palatal prosthetic devices constitutes the current state of the art of cleft palate-management. 45% of patients receiving surgical palatal correction continue to suffer from hearing loss and speech defects. Cleft palate and cleft lip is caused by interference in palatal shelf elevation, fusion or attachment during the growing stages of the fetus. Many factors contribute to cleft conditions, most common among them being heredity, prenatal nutrition, drug exposure, in combination with syndromes like Down's and Pierre Robin Syndrome as well as other genetic and environmental factors (WHO, 2002).

Palatoplasty combined with repeated ventilation tube insertion within the first year of birth has been the treatment of choice by far, greatly reducing the incidence of speech problems and otologic problems. In adults, with persistent palatal defects and speech problems, prosthetic aids like palatal lift prostheses and speech bulbs have displayed improvement in speech and swallowing. However, it is universally known that in spite of self-correcting ability of the Eustachian tube malfunction and surgical repair of cleft palate, middle ear problems and speech defects continue to exist into adolescence in about 45% of cases. Moreover, repeated insertion of prophylactic ventilation tubes predisposes children to develop tympanic membrane perforations, chronic otitis media and hearing loss in the future. Therefore, a prophylactic-cum-curative treatment is desired to address the prevailing scenario of middle ear and speech defects in cleft palate adults.

An obturator in the sense of the present invention is a plate-like prosthesis, which closes an opening or defect of the maxilla. Intraoral defects leading to oronasal communication are often encountered in the maxilla, not only resulting from congenital malformations but also from oronasal fistulation or imperative surgical removal of neoplasms. Although surgery is the mainstay for rehabilitation of such defects, it is still possible to improve the patient's social and psychological well-being with prosthodontic rehabilitation using obturators, if the patient cannot get surgical treatment at the appropriate time due to inaccessibility or economic constraints.

Currently, simple and modified obturators cast in an acrylic material are used in adults for closing the palatal defect. A palatal lift prosthesis is a kind of obturator advised in cases of velopharyngeal incompetence. It is a removable rigid plate proposed to improve speech by keeping the velum in an elevated position and holding it against the posterior pharyngeal wall at all times. Based on the mechanism of function, it potentially interferes with the production of nasal sounds and nasal breathing. Another appliance currently used is the speech bulb obturator, also known as a speech aid appliance. It is a removable device used for the treatment of velopharyngeal insufficiency by filling in the pharyngeal space. However, breathing and sleeping are potentially hampered by the bulb in place and may cause sleep apnea. It is argued that rigid acrylic resin in particular in the region of the posterior pharyngeal wall may predispose the speaker to tissue irritation or discomfort and gag reflex due to heavy weight and extension into the oropharynx. The fabrication of these devices is often associated with cumbersome nasopharyngeal impressions. None of these appliances provide a definitive solution for the above discussed chronic ear problems and speech problems. Hence, further research is imperative in developing a more patient-friendly and curative device substituting the physiologic soft palate functions and preventing chronic ear problems.

In particular, it would be highly desirable to provide a palatal obturator with the option of implantation that provides the basic functionality also of the soft palate, which is to actively close off the nasal cavities from the laryngeal pathway during swallowing in order to avoid misdirection of saliva or food into the nasal cavities while leaving the pathway open during normal breathing and speaking.

It is thus an object of the present invention to create an obturator as well as a palate implant that provides on the one side the functionality of closing the defects and mimicking the swallowing-action of the soft palate and on the other side the possibility to build up and train the palatal musculature.

To solve this object the inventive obturator for covering a defect of the hard palate and soft palate comprises a hard palate-replacing rigid plate for covering the defect of the hard palate and is characterized in that an inflatable, in particular semi-inflated soft member with a flexible outer shell is arranged adjacent to the rigid plate and having a soft palate-replacing portion extending beyond a palate bone-replacing end of the rigid plate. By this specific arrangement of a rigid plate or hard plate and a soft, inflatable member next to the rigid plate it becomes feasible to provoke the specifically designed soft member to change its shape and hardness in the region of the soft palate by the action of the tongue when swallowing, thereby closing off the nasal cavities from the laryngeal pathway. This is done by the soft member being pressed against the rigid plate by a patient's tongue during the natural swallowing process. This pressure is then passed on to the rear portion extending beyond the palate bone-replacing end of the rigid plate, i.e. the rear end of the rigid plate and reaching into the velopharyngeal region when the inventive obturator is inserted into place. When appropriately designed, the soft member can change its shape and orientation upon this increase in pressure in the rear region of the soft member and it becomes feasible to thereby close off the nasal cavities much like it would be closed off by the natural velum and the uvula of the soft palate. The region of the soft member adjacent to the rigid plate can thus be addressed as an actuation portion of the soft member and the portion of the soft member extending beyond the palate bone-replacing end of the rigid plate, i.e. the rear portion of the soft member can be addressed as the functional portion.

According to a preferred embodiment of the present invention the outer shell is made of a non-elastic material, in particular of plastic and/or silicon biocompatible material, such as polycaprolactone or polyethylene, and is shaped to mimic the shape of the soft palate during swallowing when inflated. This is preferred when the shape of the soft member can be designed to closely mimic the closed velum- and uvula-shape, since the non-elastic material allows for precise specific closure in cooperation with the corresponding anatomical structures on the posterior pharyngeal wall. The shape of the soft member and in particular its shape in the functional portion in a fully inflated state should be close to the shape of the velum and uvula while swallowing and can be determined by an individual nasopharyngeal 3D-scan of a human swallowing. The soft member is then modeled and manufactured according to this scan by an additive manufacturing process (3D printing or moulding). In this case, the outer shell is preferably made of a 3D printable material.

When the design of the outer shell is less precise and in particular when not perfectly customized to the individual patient's anatomy of his posterior pharyngeal wall, another strategy can be pursued and the outer shell is made of an elastic material, in particular silicone-material, and is shaped to mimic the shape of the soft palate during swallowing when inflated, which is in accordance with a preferred embodiment of the present invention. Providing an elastic outer shell allows for close abutment of a less specifically formed velum- and uvula-replacement by the soft member.

The major function of this appliance and also of the implantable version discussed below is the production of intelligible speech, prevention of hypernasality of vowels, prevent regurgitation of food into the nasal cavity, and improve swallowing. The implantable version additionally targets the etiology of middle ear infections and hearing loss in congenital cleft palate patients, which results from improper drainage of the Eustachian tube. Thus, the chance of hearing loss will be greatly reduced.

In order to provide large enough a volume of displacement from the actuation portion of the soft member, the soft member has a reduced inflatable volume-zone in the soft palate-replacing portion with reduced inflatable volume as compared to the inflatable volume in the portion adjacent to the rigid plate which is in accordance with a preferred embodiment of the present invention. This means that there is somewhat more volume usually of gas or fluid, that will be compressed by the tongue upon swallowing than will fit into the rear portion of the soft member, so that the rear portion will almost certainly be fully inflated thus gaining its shape defined by the outer shell.

In further developing this idea of different inflatable volumes in different regions or portions of the soft member, the invention is preferably devised such that the soft member has a reduced inflatable volume-zone in a portion between the soft palate-replacing portion and the portion adjacent to the rigid plate with reduced inflatable volume as compared to the inflatable volume in the portion adjacent to the rigid plate. This means that the middle region or portion of the soft member will consume very little volume of displacement during compression of the actuation portion of the soft member, so that very little volume for inflating the functional portion of the soft member is lost in the middle portion.

Further, a reduced inflatable volume-zone is formed by at least one preferably curved tube, preferably formed by a stabilized canal or an array of preferably curved tubes for conducting gas or fluid from the portion adjacent to the rigid plate into the soft palate-replacing portion as it is in accordance with a preferred embodiment of the present invention. This is a simple way to eliminate void volumes and direct pressure only to regions where inflation is desired.

In continuation of using tubes to influence the behavior of the soft palate-replacing portion, the soft palate-replacing portion is devised as a plurality of preferably communicating tubes embedded in a common elastic body, as it is in accordance with a preferred embodiment of the present invention. The common elastic body can be designed to the desired shape but with a flexibility that allows for it to hang down like the relaxed soft palate and uvula of a person without any defect. Upon pressure application on the palate bone-replacing portion of the soft member, the tubes embedded in the common elastic body make the soft palate-replacing portion more rigid thus elevating it to the position of occlusion in abutment to the posterior pharyngeal wall and thereby closing off the nasopharynx during swallowing.

In a preferred embodiment of the present invention, the rigid plate has means for fixing it to an anatomical structure of the body, in particular clasps for fixing it to teeth. This is known in the art of manufacturing rigid obturators and works fairly well.

In order to arrange the soft member adjacent to the rigid plate so that it is compressed by the action of the tongue while swallowing, the soft member is designed to connect to connection means of the rigid plate, in particular to magnetic elements, as it is in accordance with a preferred embodiment of the present invention. This allows for daily hygienic routines and for replacement of the soft member when worn out.

Another approach of holding the inventive obturator in place is pursued, when the obturator is implanted into the patient's body and covered by mucosa to integrate the obturator for an indefinite time. To achieve this, the rigid plate and/or the soft member preferably has/have a biocompatible surface, that preferably is self-cleansing. A biocompatible surface will allow the obturator to be implanted, covered by mucosa and over time to be fully integrated, which would eliminate the psychological strain of carrying a prosthesis in the mouth.

In order to shape the inventive obturator properly and in particular according to information gathered in a 3D-scan, the rigid plate and/or the soft member is/are produced by an additive manufacturing process as it is in accordance with a preferred embodiment of the present invention.

According to a preferred embodiment of the present invention, the rigid plate consists at least partially of additively manufactured porous titanium. Porous titanium allows for complete ossintegration of the rigid plate as an implant and it can be produced with known 3D-printing-technology to a high standard of precision.

According to a separate aspect of the invention, the inventive palate implant is made of a biocompatible collagen pad or membrane for covering a defect of the soft palate, wherein the pad or membrane at least in a boundary area thereof has a reinforcement structure of a biocompatible filament. The collagen pad represents a material that allows for complete integration into soft tissue such as the soft palate. The collagen pad can cover the area of the defect, can be covered by mucosa and covered by connective tissue but offers relatively little stability at the primary stage after implantation. To cover up for this shortcoming, the inventive pad has a reinforcement structure in a boundary region or boundary area made of a strong, biocompatible filament, so that the pad can be sutured to the mucosa and offers primary stability right after implantation before integration with connective tissue and mucosa has strengthened the implant. The biocompatible filament can be very much like suture material.

In order to allow for the pad to be fully integrated into the surrounding tissues, in particular by musculo-skeletal tissue, the pad or membrane preferably has a porous structure of collagen so that the mentioned tissues can grow into the implant.

Preferalby, the collagen pad may have an anti-scar or anti-fibrosis coating.

Preferably, the collagen pad may have a coating for expediting wound healing.

The inventive implantable obturator can be designed to behave even more like a natural, healthy soft palate, when the reinforcement structure, in addition, is designed to mimic muscle- and/or tendon-structures of the soft palate, as it is in accordance with a preferred embodiment of the present invention. The reinforcement structure thus adds stability in those regions of the implantable obturator, where the forces that are applied by muscles and tendons are directed through a healthy soft palate and will hence foster a more natural behavior of the implanted pad or membrane.

According to a preferred embodiment of the present invention, the palate implant has electrodes arranged for intermittent stimulation of muscle portions, an preferably the eustachian tube, of a patient. The same can preferably be the case for the inventive obturator. In particular M. tensor veli palatini and M. levator veli palatini are usually more or less disassembled and dystrophied in patients having cleft palate since these muscles lack their counter bearing and thus are not functional and thus not appropriately trained. The inventive implant and/or obturator with electrodes not only allows to give said muscles the structural antagonism they require to function properly, i.e. lifting the tensor veli palatini and tensioning it, thereby also opening the Eustachian tube for middle ear pressure balance but also gives the possibility to train these muscles without the patient consciously obeying to a training routine of swallowing and like movements. This allows for rebuilding muscles almost completely degenerated in grown up patients and for avoiding muscle-degeneration in young patients at an early stage.

Preferably, the electrodes are connected to a control unit, which preferably is implantable into the temporal bone (os temporale), the control unit preferably being powered by an external power source. The control unit will provide the signals to the electrodes according to the desired training pattern established by the physician and can be programmed accordingly.

Alternatively, the control unit is integrated into the hard palate-replacing rigid plate of the obturator, the control unit preferably being powered by a wireless supply parallel to a wireless rechargeable battery.

According to a preferred embodiment, it is further envisaged that the control unit is integrated into the hard palate-replacing rigid plate of the obturator and includes a coil for the power supply of the control unit. The coil is preferably used to receive the stimulation parameters for the stimulation and to charge a battery. In another embodiment, the coil is used for a permanent connection for the data transfer and power supply, if there is less space to attach a rechargeable battery into the obturator.

According to a preferred embodiment, the control unit is connected to sensor electrodes in the palate implant and/or in the inventive obturator to record an electromyogram. This configuration enables closed circuit monitoring of the training success by the electrostimulation of the palatal musculature that can be effected by the electrodes.

Based on the electromyography signals the training and function can be expanded with a feedback-training, by analyzing the measurable low muscle activity and triggering the electrostimulation synchronous to the muscle activity. Based on this feedback-stimulation the time sequences of the muscle activity can be gained in their activation by electrostimulation.

According to a preferred embodiment of the present invention, the control unit is adapted to alter the intermittent stimulation based on data derived from the electromyogram. The control unit is thus adapted to carry out an adaptive program to optimize training of the palatal muscles by electrostimulation.

The invention will now be exemplified in more detail by way of an exemplary embodiment depicted in the drawing, in which
Fig. 1a and Fig. 1b show a typical anatomical situation of a cleft palate-patient in comparison to normal anatomy of the palate,
Fig. 2a simplified sagittal sectional view of the cleft palate anatomy,
Fig. 3a simplified side elevational view of an obturator according to the present invention,
Fig. 4a and Fig. 4b are schematic sectional side views of a soft member of the inventive obturator in the relaxed state and in the compressed, hence functional state,
Fig. 5a to Fig. 5c are schematic representations of different modes of placement of the inventive obturator and
Fig. 6 is a schematic representation of an inventive palate implant.

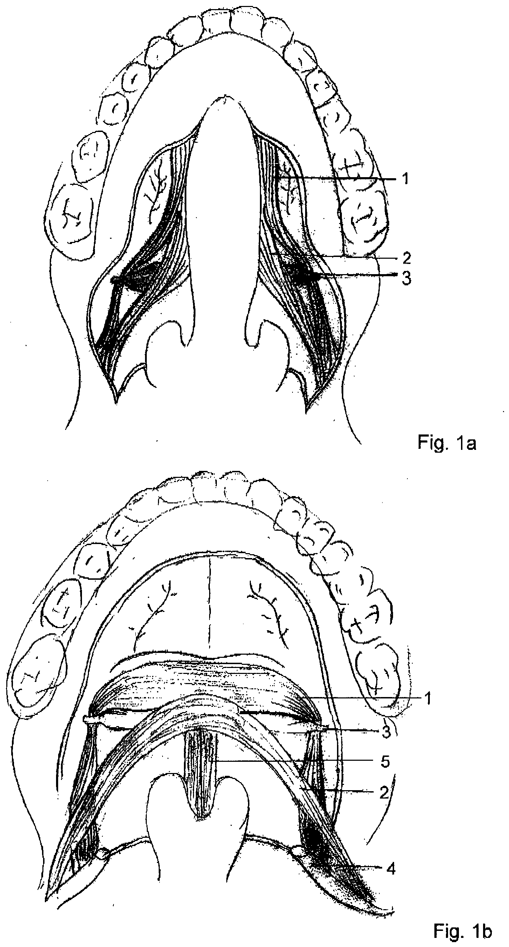

In Fig. 1a and 1b, reference numeral 1 denotes the Musculus tensor veli palatini, reference numeral 2 denotes the Musculus levator veli palatini and reference numeral 3 denotes the hamulus. Fig. 1a depicts the situation of a cleft palate, where the muscles 1 and 2 have altered attachment, do not form aponeurosis and hence lack an appropriate counter bearing. For this reasons, the muscles 1 and 2 are prone to lack proper function in cleft palate-patients. In Fig. 1b, the palatal muscles and tendons are shown in a healthy situation with the maxilla not drawn. The right and left Musculus tensor veli palatini 1 and Musculus levator veli palatini 2 form palatal aponeurosis in the middle and operate to tense and lift the soft palate. The Eustachian tube is denoted with reference numeral 4 and it can be seen that Musculus tensor veli palatini 1 also engages the Eustachian tube and opens it in order to foster pressure balance in the middle ear. Reference numeral 5 represents Musculus uvulae.

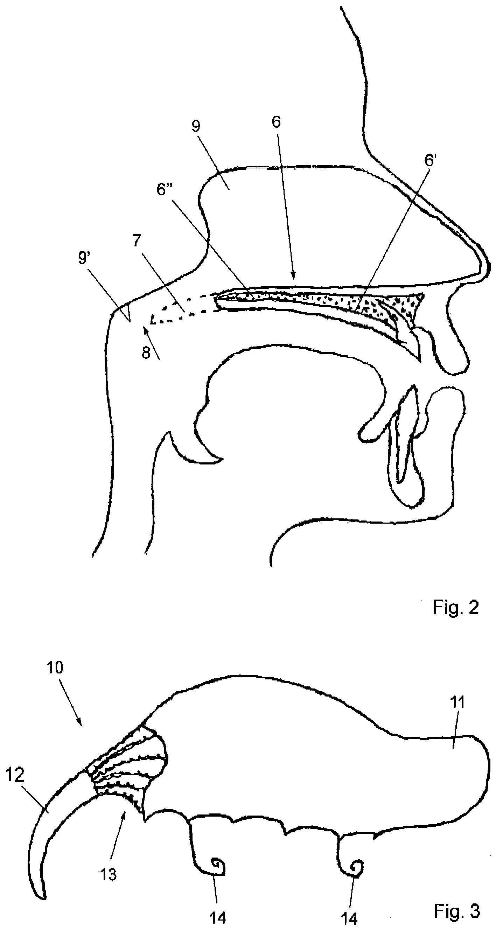

In Fig. 2 it can be seen that the palate consists basically of a hard palate, comprising the palatine bone 6, and a soft palate 7. The palatine bone 6 is formed anteriorly by the palatine process of the maxilla, referred to by 6', and posteriorly by the horizontal plate of the palatine bone, referred to by 6". When swallowing, the soft palate is lifted in the direction of the arrow 8 in order to close off the nasal cavities 9 by abutment against the posterior pharyngeal wall 9'.

The idea of the inventive obturator, which is denoted by reference numeral 10 in Fig. 3, is to cover the defect in the hard palate 6 with a rigid plate 11 and to mimic the functionality of the soft palate 7 by a soft palate replacing portion 12 of the soft member 13 that is arranged adjacent to the rigid plate 11 as will be understood in more detail in the following. The rigid plate in Fig. 3 can be fixed in place by clasps 14 that are adapted to attach to the buccal portion of teeth.

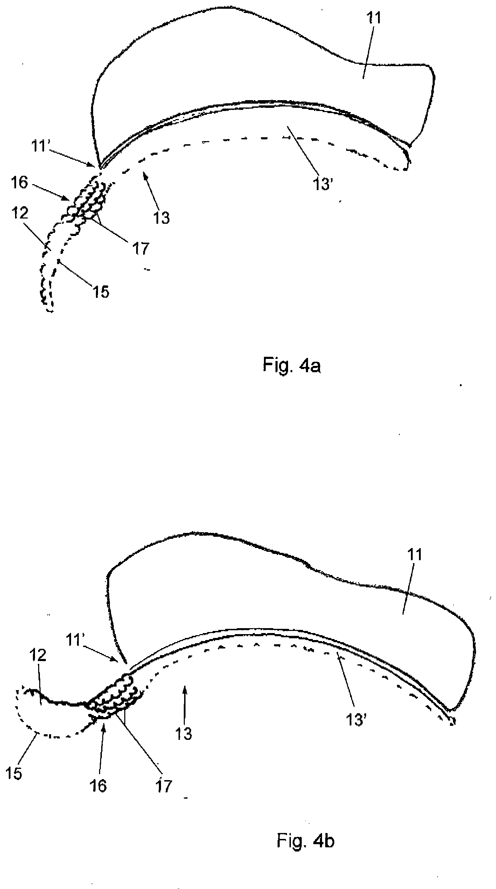

In Fig. 4a and Fig. 4b it can be seen that the soft member 13 has a portion 13' adjacent to the rigid plate 11 and a soft palate replacing portion 12 extending beyond the rear end 11' of the rigid plate 11. In Fig. 4a the soft member 13 is shown in a state where there is no pressure applied by the tongue on the portion 13'. When the tongue exerts pressure on the portion 13', the portion 13' is compressed (Fig. 4b) and the rise in pressure of a fluid or gaseous medium present in the portion 13' is passed on to the soft palate replacing portion 12 either by a flow of fluid into the portion 12 or by other ways of passing on pressure, such as, for example, displacement of a membrane or the like. Consequently, the pressure in the portion 12 will increase, the portion 12 will therefore assume its fully inflated shape, which is determined by the design of the outer shell 15 of the soft member 13. The soft palate replacing portion 12 is lifted up and is able to occlude the passage to the nasal cavities by abutment against the posterior pharyngeal wall (not shown in Fig. 4a and Fig. 4b). Reference numeral 16 denotes a reduced inflatable volume zone in a portion between the soft palate-replacing portion 12 and the portion 13' with reduced inflatable volume as compared to the inflatable volume in the portion 13'. The reduced inflatable volume in the zone 16 is achieved by restricting the inflatable volume to curved tubes 17.

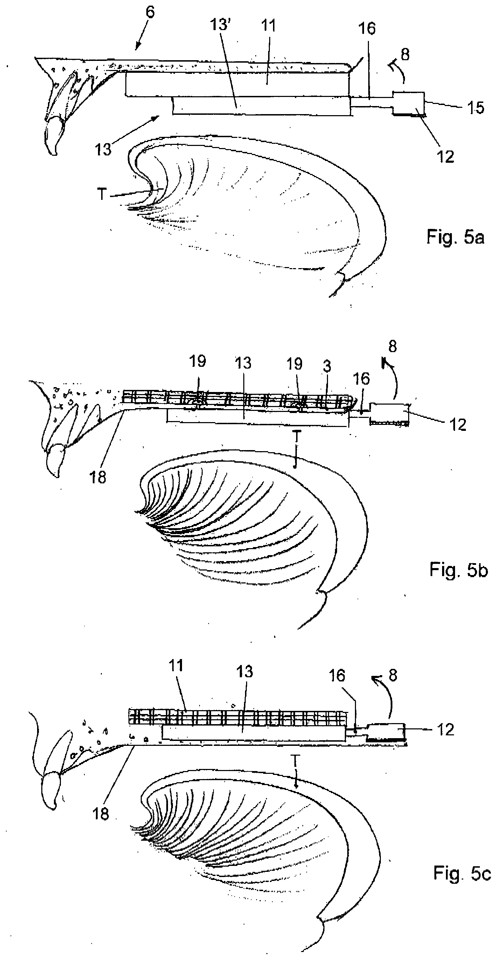

Fig. 5a illustrates a situation where neither the rigid plate 11 nor the soft member 13 is implanted into the patient's palate. In this situation the rigid plate 11 is merely fixed in place, for example by clasps 14 as shown in Fig. 3 to cover the defect in the hard palate 6. The tongue T will compress the actuation portion 13 in the swallowing process thereby inflating the soft palate-replacing portion 12, which will then assume its elevated conformation. This basic principle is repeated in Fig. 5b and Fig. 5c. In Fig. 5b, however, the rigid plate 11 is implanted under the mucosa 18 and the soft member 13 is attached to the rigid plate 11 by implanted pins 19. In Fig. 5c, both the rigid plate 11 and the soft member 13 are implanted under the mucosa 18.

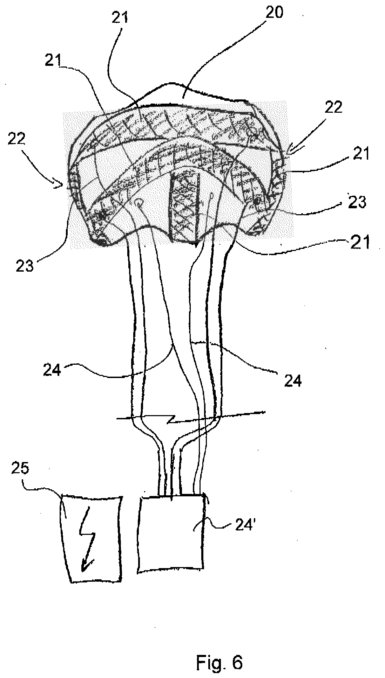

The palate implant according to Fig. 6 consists of a pad 20 or membrane 20 of 3D-printed collagen mesh that has a reinforcement structure 21 on a boundary area 22 thereof. The reinforcement structure 21 is made of a biocompatible filament and, in the example of Fig. 6 is also designed to mimic muscle-structures as explained in the context of Fig. 1b. The pad is further equipped with stimulation electrodes 23 in locations adapted to influence the palatal muscles discussed with reference to Fig. 1a and Fig. 1b. Further, the implant has a set of sensor electrodes 24 that are connected to the control unit 24' that also controls the electric potential of the electrodes 23. The control unit 24' cooperates wirelessly with an external power source 25 that can, for example be placed behind a patient's ear.

## Claims

1. Obturator for covering a defect of the hard palate and soft palate, wherein the obturator comprises a hard palate-replacing rigid plate for covering the defect of the hard palate, **characterized in that** an inflatable, in particular semi-inflated soft member with a flexible outer shell is arranged adjacent to the rigid plate and having a soft palate-replacing portion extending beyond a palate bone-replacing end of the rigid plate.

2. Obturator according to claim 1, **characterized in that** the outer shell is made of a non-elastic material, in particular of plastic and/or silicone biocompatible material, and is shaped to mimic the shape of the soft palate during swallowing when inflated.

3. Obturator according to claim 1, **characterized in that** the outer shell is made of an elastic material, in particular silicone-material, and is shaped to mimic the shape of the soft palate during swallowing when inflated.

4. Obturator according claim 1, 2 or 3, **characterized in that** the soft member has a reduced inflatable volume-zone in the soft palate-replacing portion with reduced inflatable volume as compared to the inflatable volume in the portion adjacent to the rigid plate.

5. Obturator according to any one of claims 1 to 4, **characterized in that** the soft member has a reduced inflatable volume-zone in a portion between the soft palate-replacing portion and the portion adjacent to the rigid plate with reduced inflatable volume as compared to the inflatable volume in the portion adjacent to the rigid plate.

6. Obturator according to claim 4 or 5, **characterized in that** a reduced inflatable volume-zone is formed by at least one preferably curved tube, preferably formed by an array of preferably curved tubes for conducting gas or fluid from the portion adjacent to the rigid plate into the soft palate-replacing portion.

7. Obturator according to any one of claims 1 to 6, **characterized in that** the soft palate-replacing portion is devised as a plurality of preferably communicating tubes embedded in a common elastic body.

8. Obturator according to any one of claims 1 to 7, **characterized in that** the rigid plate has means for fixing it to an anatomical structure of the body, in particular clasps for fixing it to teeth.

9. Obturator according to any one of claims 1 to 8, **characterized in that** the soft member is designed to connect to connection means of the rigid plate, in particular to magnetic elements.

10. Obturator according to any one of claims 1 to 9, **characterized in that** the rigid plate and/or the soft member has/have a biocompatible surface.

11. Obturator according to nay one of claims 1 to 10, **characterized in that** the rigid plate and/or the soft member is/are produced by an additive manufacturing process.

12. Obturator according to one of claims 1 to 11, **characterized in that** the rigid plate consists at least partially of additively manufactured porous titanium.

13. Palate implant, in particular for use in an obturator according to any one of claims 1 to 12, made of a biocompatible collagen pad or membrane for covering a defect of the soft palate, wherein the pad or membrane at least in a boundary area thereof has a reinforcement structure of a biocompatible filament.

14. Palate implant according to claim 13, **characterized in that** the pad or membrane has a porous structure.

15. Palate implant according to claim 13 or 14, **characterized in that** the reinforcement structure, in addition, is designed to mimic muscle- and/or tendon-structures of the soft palate.

16. Palate implant according to claim 13, 14 or 15, **characterized in that** the palate implant has electrodes arranged for intermittent stimulation of muscle portions of a patient.

17. Palate implant according to any one of claims 13 to 16, **characterized in that** the electrodes are connected to a control unit, which preferably is implantable into the temporal bone (os temporale), the control unit preferably being powered by an external power source.

18. Palate implant according to any one of claims 13 to 17, **characterized in that** the control unit is connected to sensor electrodes in the palate impact to record an electromyogram.

19. Palate implant according to any one of claims 13 to 18, **characterized in that** the control unit is adapted to alter the intermittent stimulation based on data derived from the electromyogram.

20. Palate implant according to any one of claims 13 to 19, **characterized in that** collagen pad is produced by an additive manufacturing process.
